# FASCICULE DE BREVET EUROPEEN

(11) **EP 0 523 186 B1**
(45) Date de publication et mention de la délivrance du brevet: **03.07.1996**
(21) Numéro de dépôt: 91908625.6
(22) Date de dépôt: 05.04.1991
(51) Int. Cl.: C07H 13/06, C12P 19/04

(54) **SUBSTANCES DE STRUCTURE LIPO-OLIGOSACCHARIDIQUE PROPRES A JOUER LE ROLE DE SIGNAUX SYMBIOTIQUES PLANTES-SPECIFIQUES, LEURS PROCEDES DE PRODUCTION ET LEURS APPLICATIONS**
LIPO-OLIGOSACCHARIDE ALS PFLANZENSPEZIFISCHES REGULIERENDES MITTEL, VERFAHREN ZU IHRER HERSTELLUNG UND IHRE VERWENDUNGEN
SUBSTANCE WITH LIPO-OLIGOSACCHARIDE STRUCTURE CAPABLE OF ACTING AS PLANT-SPECIFIC SYMBIOTIC SIGNALS, PROCESSES FOR PRODUCING THEM AND THEIR APPLICATIONS

(30) Priorité: 06.04.1990 FR 9004764
(43) Date de publication de la demande: 20.01.1993
(73) Titulaire: CENTRE NATIONAL DE LA RECHERCHE SCIENTIFIQUE (CNRS), 75794 Paris Cédex 16 (FR); INSTITUT NATIONAL DE LA RECHERCHE AGRONOMIQUE, F-75341 Paris Cédex 07 (FR)
(72) Inventeur: LEROUGE, Patrice, F-31300 Toulouse (FR); ROCHE, Philippe, F-31400 Toulouse (FR); FAUCHER, Catherine, F-32600 Sefoufielle (FR); MAILLET, Fabienne, F-31450 Pompertuzat (FR); DENARIE, Jean, F-31320 Castanet-Tolosan (FR); PROME, Jean-Claude, F-31320 Pechbusque (FR); TRUCHET, Georges, F-31450 Ayguesvives (FR)
(74) Mandataire: Ores, Irène
(86) Numéro de dépôt international: FR9100283
(87) Numéro de publication internationale: WO9115496

(56) Documents cités:
- WO-A-87/06796
- DE-A- 3 735 365
- CHEMICAL ABSTRACTS, vol. 110, no. 25, 19 juin 1989, Columbus, OH (US); p. 689, no. 232023c/
- CHEMICAL ABSTRACTS, vol. 102, no. 3, 21 janvier 1985, Columbus, OH (US); M. ABE et al., p. 416, no. 21445p/
- NATURE, vol. 344, 19 avril 1990; P. LEROUGE et al., pp. 781-784/

## Description

La présente Invention est relative à des substances de structure lipo-oligosaccharidique aptes à constituer des signaux symbiotiques plantes-spécifiques, à des procédés de production de ces substances et aux applications de celles-ci.

Comme on le sait, les végétaux ont besoin, pour leur croissance d'une source d'azote combiné comme l'ammoniaque ou le nitrate. La fixation de l'azote, par réduction chimique ou biologique de l'azote atmosphérique (N₂) en ammoniaque (NH₃), joue donc un rôle essentiel dans la production agricole.

Comme on le sait également, des micro-organismes symbiotiques peuvent favoriser la croissance et le développement des plantes par fixation biologique de l'azote.

Les Rhizobiacées sont des bactéries du sol Gram-négatives qui fixent généralement l'azote en association symbiotique avec des végétaux : l'établissement d'une telle symbiose avec des bactéries fixatrices d'azote permet à de nombreuses espèces végétales de croître sur des sols pauvres en azote assimilable. Grâce à la photosynthèse, le partenaire végétal fournit à la bactérie l'énergie nécessaire à la réduction de l'azote moléculaire en ammoniaque. En retour, l'ammoniaque fixé par le microsymbionte est fourni à la plante-hôte qui l'intègre dans son métabolisme azoté. L'association symbiotique qui s'établit entre des bactéries fixatrices d'azote, telles que les Rhizobiacées, et des plantes de la famille des Légumineuses est la plus importante sur le plan écologique et agronomique. Cette association se traduit par la formation de nodosités, ou nodules, essentiellement sur les racines des plantes-hôtes. A l'intérieur de ces nodosités, les bactéries réduisent l'azote atmosphérique en ammoniaque par l'intermédiaire du complexe enzymatique de la nitrogénase.

La symbiose entre les bactéries fixatrices d'azote de la famille des Rhizobiacées (genres *Rhizobium, Bradyrhizobium, Sinorhizobium,* et *Azorhizobium)* et les plantes de la famille des Légumineuses joue donc un rôle très important en agriculture tempérée et tropicale. Parmi ces plantes on citera notamment des oléoprotéagineux comme le soja et l'arachide, des fourrages comme la luzerne et le trèfle, des protéagineux comme le pois et la féverole, des plantes vivrières comme les haricots, pois, lentilles et pois chiches, des engrais verts comme *Sesbania* etc... Grâce à ces symbioses la culture des Légumineuses est souvent moins coûteuse en engrais azotés que la culture des plantes appartenant à d'autres familles. A cet égard, il convient de rappeler que l'utilisation massive des engrais azotés présente un certain nombre d'inconvénients. Tout d'abord la synthèse, le transport et l'épandage des engrais, est coûteuse en énergie fossile, ce qui a plusieurs conséquences : au niveau de l'exploitation agricole elle augmente les coûts de production des agriculteurs et au niveau de l'environnement elle contribue à l'effet de serre par accroissement de la teneur en CO₂. Par ailleurs, une fertilisation azotée non raisonnée ou excessive entraîne une pollution des eaux continentales avec une eutrophisation des eaux de surface et une augmentation de la teneur en nitrates des nappes phréatiques. Ces diverses raisons militent en faveur d'une utilisation accrue de la fixation biologique de l'azote.

Etant donné les conséquences très préjudiciables de l'utilisation excessive d'engrais azotés, il convient donc d'accroître la contribution de la fixation biologique d'azote par les plantes et notamment par les espèces cultivées qui interviennent pour une part importante dans l'alimentation humaine et animale. La solution la plus acceptable, tant du point de vue écologique que du point de vue économique, est l'amélioration de la symbiose Rhizobiacées-Légumineuses.

Il a été proposé de réaliser cette amélioration par un apport de Rhizobiacées (notamment *Rhizobium* ou *Bradyrhizobium)* au moment du semis, soit par enrobage des graines, soit à l'aide de granulés mélangés aux graines, soit à l'aide d'une culture en milieu liquide. Ces apports bactériens ne sont cependant efficaces que dans les cas, relativement rares, où les bactéries symbiotiques appropriées sont naturellement absentes ou peu abondantes dans les sols. Dans les cas contraires, c'est à dire dans les sols contenant déjà ces bactéries, il est pratiquement impossible d'imposer une souche introduite volontairement, en raison de la compétition avec les bactéries indigènes contenues dans les sols, qui, même si elles ne sont pas forcément efficaces pour la fixation de l'azote, n'en constituent pas moins un facteur limitant pour l'introduction de bactéries sélectionnées.

Il a également été proposé de favoriser la symbiose Rhizobium-Légumineuses en traitant les plantes par un exopolysaccharide dérivé de bactéries du genre *Rhizobium* ou par un oligosaccharide contenant un ou plusieurs motifs d'un tel exopolysaccharide (EPS) - cf. la demande internationale PCT publiée sous le N° WO 87/06796 déposée au nom de THE AUSTRALIAN NATIONAL UNIVERSITY et mentionnant comme Inventeurs : B.G.ROLFE, S.P. DJORDJEVIC, J.W. REDMOND et M. BATLEY -. Toutefois, cet EPS est un produit codé par des gènes non symbiotiques. En effet la biosynthèse de ces exopolysaccharides n'est pas sous le contrôle direct des gènes *nod* qui contrôlent l'infection et la nodulation. Ces exopolysaccharides sont synthétisés par des souches de *Rhizobium* qui ont été guéries de leur plasmide *pSym,* plasmide qui porte l'essentiel des gènes symbiotiques et notamment les gènes *nod.*

Les gènes impliqués dans le processus de formation des nodules ont été localisés et plusieurs gènes de nodulation (gènes *nod)* communs et spécifiques ont été identifiés et caractérisés (voir Long, S. R., Cell, 1989,56,203-214). Alors que les gènes *nodA,B,C* sont des gènes de nodulation communs aux différentes espèces de Rhizobiacées symbiotiques, il existe des gènes *nod* spécifiques qui déterminent le spectre d'hôte et qui varient, de ce fait, chez les différentes espèces, et des gènes régulateurs, de type *nod*D*,* qui contrôlent l'expression de l'ensemble des gènes nod.

Les gènes communs *nod*A,B,C ont été mis en évidence dans les quatre genres bactériens capables d'établir une symbiose fixatrice d'azote avec les Légumineuses : *Rhizobium, Bradyrhizobium, Sinorhizobium,* et *Azorhizobium.* Pour le genre *Rhizobium* la séquence nucléotidique de ces gènes a été réalisée chez *R.meliloti* (Török et al., Nucleic Acids Res., 1984,12,9509-9522 ; Jacobs et al., J. Bactériol., 1985,162,469-476 ; Egelhoff et al., DNA, 1985,4,241-242), *R.leguminosarum* (Rossen et al., Nucl. Acids Res., 1984,12,9497-9508), *R.trifolii* (Schofield et al., Nucl. Acids Res., 1986,14,2891-2903).

Pour Bradyrhizobium sp. (Scott, Nucl. Acids Res., 1986,14,2905-2919).

Pour *Azorhizobium caulinodans* (Goethals et al., Mol. Gen. Genet., 1989,219,289-298).

Une équipe de Chercheurs comprenant plusieurs des Inventeurs de la présente Invention a montré que chez *Rhizobium meliloti*, les gènes communs *nod*A,B,C induisent, conjointement avec les gènes spécifiques *nod*H et *nod*Q, la production de signaux Nod extra-cellulaires hôte-spécifiques, présents dans les surnageants de culture de ces bactéries : cf FAUCHER et al., J. BACTERIOL (1988), 172, 5489-5429 et FAUCHER et al. Molec. Plant-Microbe Interact. (1989), 2, 291-300, entre autres. La seconde de ces deux Publications rend compte, en outre, d'un fractionnement du surnageant stérile par ultrafiltration, qui a permis de mettre en évidence la présence de deux facteurs Nod de masse moléculaire apparente inférieure à 5 000 Da.

La spécificité d'infection et de nodulation est déterminée, chez *R.meliloti,* sur deux plans, à savoir : - les gènes *nodD* activent l'expression d'autres opérons *nod* en fonction de la présence de signaux spécifiques produits par les plantes (Gyorgypal et al., Molec. Gen. Genet., 1988,212,85-92) et, - des gènes spécifiques tels que *nodH* et *nodQ*, lorsqu'ils sont activés, déterminent la production de signaux extra-cellulaires bactériens (facteurs Nod), qui permettent la reconnaissance et la stimulation d'une légumineuse-hôte comme la luzerne (Faucher et al., J. Bactériol, 1988,172,5489-5499 ; Faucher et al., Molec. Plant-Microbe Interact., 1989,2,291-300). Toutefois, la structure chimique des signaux bactériens n'était pas connue.

La Demande de Brevet Japonaise 63255294 décrit des tri-N-acylchitotrioses, utilisables comme agents antitumoraux, anti - infectieux, hypolipémiants, anti-bactériens et régulateurs de la croissance des plantes.

Ce document ne mentionne ou ne suggère aucune activité potentielle de ces molécules sur la symbiose Rhizobiacées-Légumineuses.

La présente Invention s'est donné pour but de définir la structure chimique de signaux Nod symbiotiques, et de pourvoir à des substances présentant une telle structure chimique, de pourvoir également à des procédés de production de substances aptes à jouer le rôle de tels signaux Nod symbiotiques et de proposer des applications de ces substances pour le traitement d'organismes appartenant au règne végétal.

Les Inventeurs ont obtenu des substances sensiblement pures, présentant la structure d'un facteur Nod ou de l'un de ses analogues.

On entend par "facteur Nod", au sens de la présente Invention, une molécule-signal produite sous le contrôle direct de gènes *nod,* qui est un signal grâce auquel les bactéries symbiotiques sont capables d'infecter les plantes et d'induire la formation de nodosités. Les facteurs Nod obtenus par les Inventeurs sont constitués par des lipo-oligosaccharides non dérivés des exopolysaccharides, et leur biosynthèse est sous le contrôle d'au moins un gène de nodulation (*nodA,B,C*) commun aux Rhizobiacées, en particulier aux genres *Rhizobium, Bradyrhizobium, Sinorhizobium* et *Azorhizobium.*

La présente Invention a pour objet l'utilisation d'un lipo-oligosaccharide ou d'un mélange de lipo-oligosaccharides, comprenant un oligomère d'hexosamine, dont l'hexosamine terminale non réductrice est N-substituée par un acide gras, en tant que signal symbiotique plante-spécifique, pour favoriser la symbiose Rhizobiacées-Légumineuses. Ce signal est apte à amplifier la capacité de la bactérie à infecter la plante-hôte à laquelle il est associé, et/ou à accélérer la formation de nodules sur la plante-hôte à laquelle il est associé, et/ou à induire la transcription de gènes symbiotiques de légumineuses.

Selon un mode de mise en oeuvre préféré de l'utilisation qui fait l'objet de la présente Invention, la glucosamine terminale non-réductrice de l'oligomère de N-acétyl glucosamine est N-substituée par un acide gras comprenant au moins 12 atomes de carbone.

La présente Invention a également pour objet une substance lipo-oligosaccharidique caractérisée en ce qu'elle répond à la formule générale I ci-après : 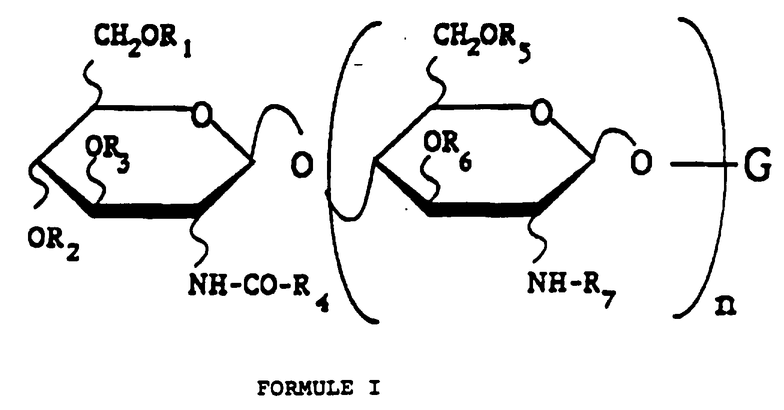 dans laquelle :
- G est une hexosamine diversement substituée, par exemple, par un groupe acétyl sur l'azote, un groupe sulfate, un groupe acétyl et/ou un groupe éther sur un oxygène,
- R₁, R₂, R₃, R₅, R₆, R₇, qui peuvent être identiques ou différents, représentent H, CH₃CO-, C_{X}H_{Y}CO- où x est un nombre entier compris entre 0 et 17, et y est un nombre entier compris entre 1 et 35, ou tout autre groupe acyl, tel que par exemple un carbamyl
- R₄ représente une chaîne aliphatique saturée ou mono-, di-, ou tri-insaturée comportant au moins 12 atomes de carbone, et
- n est un nombre entier compris entre 1 et 4. Selon un mode de réalisation préféré de la présente Invention, G représente :
   . Chez *R. meliloti* une N-acétyl-D-glucosamine 6-sulfate
   . Chez *R. leguminosarum b.v. viciae* une N-acétyl-D-glucosamine.

Selon une disposition avantageuse de ce mode de réalisation, le lipo-oligosaccharide conforme à l'Invention est caractérisé en ce qu'il répond à la formule (II) ci-après : 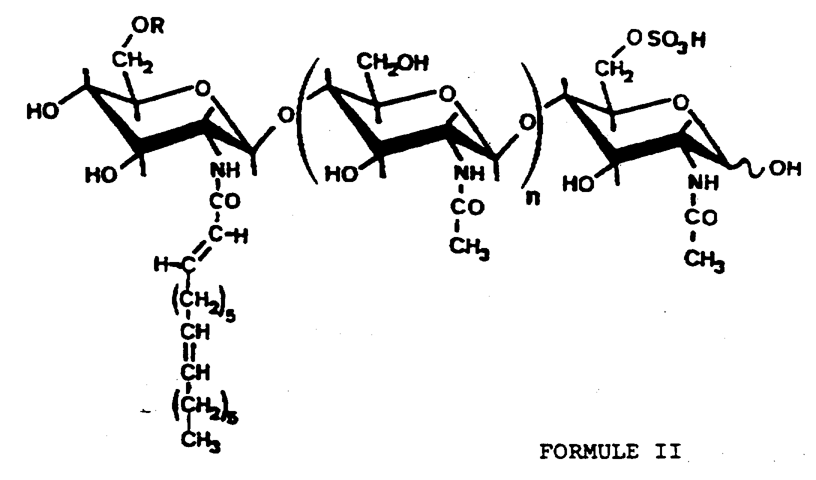 dans laquelle R représente H ou CH₃CO- et n est égal à 2 ou 3.

Les lipo-oligosaccharides de *R*. *meliloti* répondant à la formule générale (II) dans laquelle R représente H sont désignés ci-après sous le terme général NodRm ; lorsque n = 2, le lipo-oligosaccharide correspondant est dénommé NodRm-1 ; lorsque n = 3, le lipo-oligosaccharide correspondant est dénommé NodRm-3.

Les lipo-oligosaccharides répondant à la formule générale (II) dans laquelle R représente CH₃CO- sont désignés ci-après sous le terme général Ac-NodRm ; lorsque n = 2, le lipo-oligosaccharide correspondant est dénommé Ac-NodRm-1 ; lorsque n = 3, le lipo-oligosaccharide correspondant est dénommé Ac-NodRm-3.

Les facteurs Nod conformes à l'Invention peuvent être purifiés à partir de surnageants de culture de Rhizobiacées, synthétisés par les voies de synthèse séquentielle ou convergente usuelles dans les synthèses osidiques, ou produits par génie génétique, à partir de gènes *nod* clonés dans des microorganismes appartenant ou non à la famille des Rhizobiacées, éventuellement sous le contrôle d'un promoteur approprié et/ou en présence de gènes régulateurs ayant fait l'objet de mutations appropriées.

Selon un mode de réalisation avantageux d'un procédé de production de la substance sensiblement pure conforme à la présente Invention, au cours d'une première étape on produit un plasmide recombinant résultant du clonage dans un plasmide capable de se répliquer dans une bactérie Rhizobiacée ou tout autre bactérie appropriée, (1) soit d'un fragment qui contient des gènes *nod* communs et spécifiques, ainsi que des gènes régulateurs d'une bactérie Rhizobiacée donnée, soit (2) des gènes régulateurs de l'expression dés gènes *nod.* Les bactéries Rhizobiacées ou toutes autres bactéries appropriées sont ensuite modifiées par introduction dudit plasmide recombinant, pour obtenir une souche mutante très surproductrice de facteurs Nod, et on cultive ladite souche mutante dans un milieu de culture approprié ; - au cours d'une deuxième étape, on recueille le surnageant de culture que l'on purifie par extraction par un alcool inférieur approprié, ou par extraction solide-liquide suivie d'une chromatographie HPLC en phase inverse du résidu d'extraction, d'une chromatographie par perméation de gel, d'une chromatographie par échange d'ions et d'une chromatographie HPLC analytique en phase inverse.

Selon un mode de réalisation avantageux de ce procédé, le plasmide surproducteur de facteur Nod est introduit dans une bactérie Rhizobiacée mutante non-productrice d'exopolysaccharides.

Selon un autre mode de réalisation avantageux du procédé de production de la substance sensiblement pure conforme à la présente Invention, on part d'une souche sauvage de bactéries Rhizobiacées fortement productrice de facteurs Nod, que l'on cultive dans un milieu de culture approprié, après quoi l'on recueille le surnageant de culture que l'on traite selon les modalités indiquées plus haut.

La présente Invention a également pour objet un agent de traitement de plantes dont le ou un constituant actif est une substance lipo-oligosaccharidique sensiblement pure conforme à l'Invention, telle que définie plus haut, et en particulier :
- un agent de stimulation des mécanismes de défense des plantes contre les pathogènes,
- un agent de stimulation des propriétés symbiotiques des Légumineuses, notamment à l'égard de la fixation d'azote.

Selon un mode de réalisation avantageux de l'agent de traitement de plantes conforme à la présente Invention, celui-ci est formulé sous la forme d'une composition d'enrobage de graines ou d'une solution ou suspension aqueuse pour pulvérisation, dans laquelle ladite substance est présente seule ou associée à d'autres constituants actifs.

Selon un autre mode de réalisation avantageux de l'agent de traitement de plantes conforme à la présente Invention, ladite substance est présente dans les compositions d'enrobage ou les solutions ou suspensions aqueuses, à une concentration comprise entre 10⁻⁶ M et 10⁻¹⁴ M, lorsque l'agent de traitement de plantes est destiné à être utilisé comme agent de stimulation des mécanismes de défense ou des propriétés symbiotiques.

Outre les dispositions qui précèdent, l'Invention comprend encore d'autres dispositions, qui ressortiront de la description qui va suivre.

L'Invention sera mieux comprise à l'aide du complément de description qui va suivre, qui se réfère aux exemples qui vont suivre.

Il doit être bien entendu, toutefois, que ces exemples ainsi que les dessins annexés, sont donnés uniquement à titre d'illustration de l'objet de l'Invention, dont ils ne constituent en aucune manière une limitation.

### EXEMPLES

### Exemple 1 - Préparation des composés conformes à l'Invention par purification à partir d'un milieu de culture de Rhizobium meliloti.

### A. Production du composé

La production de facteurs Nod extracellulaires par *Rhizobium* est sous le contrôle d'une part des gènes communs *nodA,B,C* (van Brussel et al., J. Bacteriol, 1986,165,517-522; Zaat et al., J. Bacteriol., 1987,169,3388-3391; Faucher et al., J. Bacteriol., 1988,170,5489-5499) et d'autre part des gènes *nod* de spécifité d'hôte, par exemple chez *R.meliloti,* les gènes *nodH* et *nodQ* (Faucher et al., J. Bacteriol, 1988,170,5489-5499 ; Faucher et al., Molec. Plant-Microbe Interact., 1989,2,291-300).

La régulation de la transcription des gènes communs *nodA,B,C* est sous le contrôle de protéines régulatrices codées par les gènes *nodD1, nodD2, nodD3* et *syrM* (Mulligan et al., PNAS, 1985,82,6609-6613 ; Gyorgypal et al., Mol. Gen. Genet., 1988,212,85-92). La protéine NodD1 n'est active qu'en présence de certains flavonoïdes inducteurs, présents dans les exsudats racinaires de Légumineuses, comme la lutéoline (Mulligan et al., PNAS, 1985,82,6609-6613 ; Peters et al., Science, 1986,233,977-980). La présence des gènes *nodD3* et *syrM* sur un plasmide multicopie provoque une activation constitutive des gènes *nodA,B,C* même en l'absence de flavonoïdes inducteurs (Mulligan et al., Genetics, 1989,122,7-18).

Pour déterminer la régulation des autres gènes *nod* les Inventeurs ont construit des fusions entre les gènes de spécificité d'hôte *nodE, nodG* et *nodH* et le gène *lacZ* d'*E.coli* codant pour la β-galactosidase. Ces fusions ont permis de montrer que chez *R.meliloti* les gènes *nod* de spécificité d'hôte sont régulés de la même façon que les gènes communs *nodA,B,C*: (i) leur transcription est activée par *nodD1, nodD3* et *syrM,* (ii) la lutéoline est nécessaire pour l'activation par *nodD1,* (iii) l'activation de la transcription est beaucoup plus forte quand les gènes régulateurs et les gènes de structure sont sur un plasmide du groupe d'incompatibilité Inc-P1 (présents à 5-10 exemplaires par cellule).

Ces indications ont incité les Inventeurs à choisir un plasmide dérivé du pRK2, le pRK290, dans lequel a été cloné un fragment de 30kb de la région *nod* du mégaplasmide *pSym* de *R.meliloti.* Ce plasmide recombinant, le pGMI149 contient l'ensemble des gènes *nod* communs et spécifiques, ainsi que les gènes régulateurs *nodD1, nodD3* et *syrM* (Debellé et al., J. Bacteriol., 1986,168,1075-1086 ; Faucher et al., J. Bactériol, 1988,170,5489-5499). Ils ont montré que des souches de *R.meliloti* contenant le plasmide pGMI149 sont surproductrices de facteurs *Nod :* en effet, leur production est supérieure d'au moins cent fois par rapport à des souches de *R.meliloti* sauvages.

Pour la production à grande échelle des facteurs Nod, par exemple pour des cultures en fermenteur, la forte production d'exopolysaccharides peut présenter des inconvénients pour la filtration des cultures et pour la purification des molécules-signal. Les Inventeurs ont donc jugé préférable d'introduire le plasmide surproducteur pGMI149 dans le mutant EJ355 de *R.meliloti* qui ne produit pas d'exopolysaccharides. La souche EJ355 (pGMI149) produit en abondance les facteurs Nod mais pas d'exopolysaccharides.

En utilisant des fusions de gènes entre les gènes *nod* de *R.meliloti* et le gène *lacZ* d'*Escherichia coli* (fusions *nod::lac*) pour mesurer le niveau d'expression des gènes *nod,* les Inventeurs ont mis au point un milieu de culture qui permet une bonne expression des gènes *nod* et une production élevée de facteurs Nod. Un milieu satisfaisant est un milieu minéral qui contient du succinate comme source de carbone et du glutamate comme source d'azote et de carbone. En effet, dans un milieu riche, contenant des lysats de protéines et des extraits de levures, l'activation de la transcription des gènes *nod* est moins bonne ; en outre la purification des facteurs Nod est plus facile à partir de surnageants de cultures préparés à partir de milieux simples. La composition de ce milieu est, par exemple, la suivante :
- Sels minéraux :
   KH₂PO₄ 14,7 mM ; K₂HPO₄ 11,5 mM ; MgSO₄ 1 mM ;
   CaCl₂ 0,46 mM; FeCl₃ 37 µM,
- Composés organiques :
   L-glutamate de sodium 5,3 mM ; succinate de sodium 7,4 mM ; biotine 2 µM ; lutéoline 10 µM.

### B.1. Purification du composé

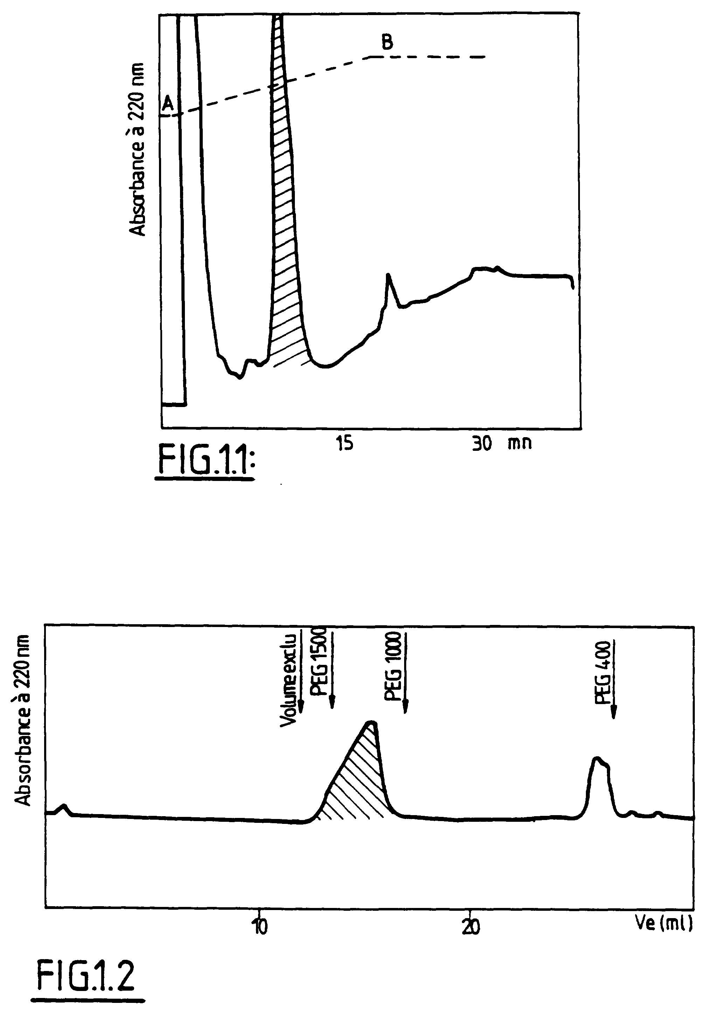

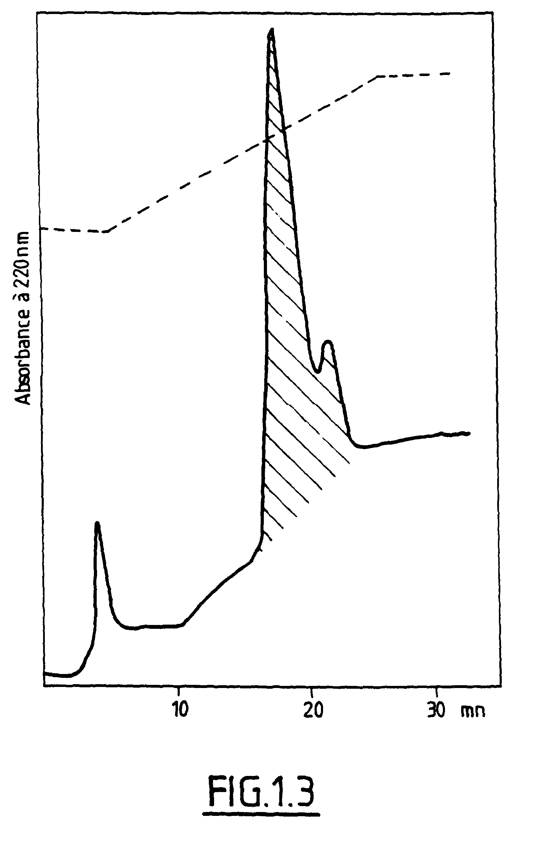

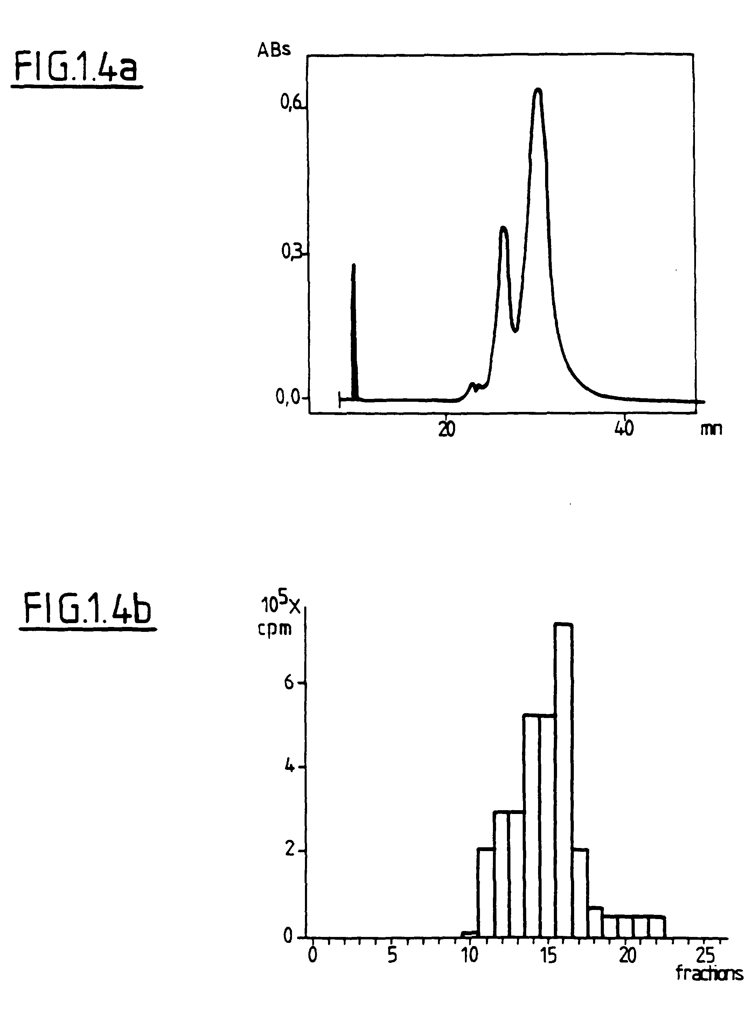

**B.1.1.** Le composé est extrait du milieu de culture de *Rhizobium meliloti* (15 l) par du butanol (2 extractions de 3 litres de BuOH). La phase butanolique est lavée à l'eau (1 litre) puis concentrée sous vide. Le résidu obtenu est dissous dans l'eau (500 ml) et extrait 2 fois par l'acétate d'éthyle (2 fois 300 ml). La phase aqueuse est enfin concentrée puis lyophilisée.
**B.1.2. Chromatographie HPLC en phase inverse**
   Le résidu obtenu par extraction butanolique est chromatographié par HPLC en phase inverse sur une colonne µ BONDAPAK® C₁₈, 10µm - Water Associates -, 7,5X250 mm, avec un débit de 2 ml/mn et en utilisant un gradient linéaire à partir d'un mélange A eau-éthanol,80-20 jusqu'à l'éthanol pur B (Fig. 1.1) ; la détection est effectuée à 220 nm. La zone présentant une activité biologique est collectée pour purifications complémentaires.
**B.1.3. Chromatographie par perméation de gel**
   La fraction est purifiée par perméation de gel sur colonne SEPHADEX® LH20 (colonne 1x20 cm ; phase LH20 Pharmacia) dans un solvant tel que l'éthanol, à un débit de 4 ml/heure. La détection est effectuée à 220 nm. La colonne a été préalablement calibrée par rapport à des témoins de polyéthylèneglycols (PEG) (cf. Fig. 1.2). La fraction hachurée active est collectée.
**B.1.4. Chromatographie par échange d'ions**
   Sur colonne d'échange d'ions (colonne DEAE-Trisacryl 1X2 cm ; IBF), le composé actif (zone hachurée) est élué par un gradient linéaire de Nacl entre 0 et 0,1 N dans un tampon Tris-HCl 5X10⁻³ N, pH=8,2 (Fig. 1.3).
**B.1.5. Chromatographie HPLC analytique en phase inverse**
   L'ultime étape de purification est une chromatographie HPLC en phase inverse sur colonne analytique (4,5x250 mm ; phase SPHERISORB®/C₁₈ 5 µm) avec, comme solvant, un mélange eau-acétonitrile 80-20 à un débit de 1ml/mn ; 300 µg sont injectés (Fig. 1.4a). Deux pics absorbant à 220 nm sont ainsi détectés et présentent une activité biologique. L'étude structurale montrera qu'il s'agit en fait du même composé apparaissant sous deux formes en équilibre (formes anomériques α et β libres).

### B.2. Analyse des constituants

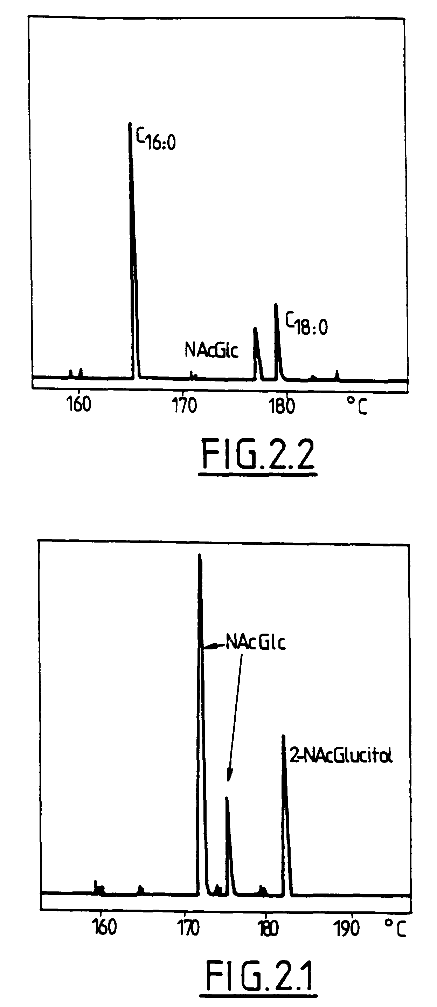

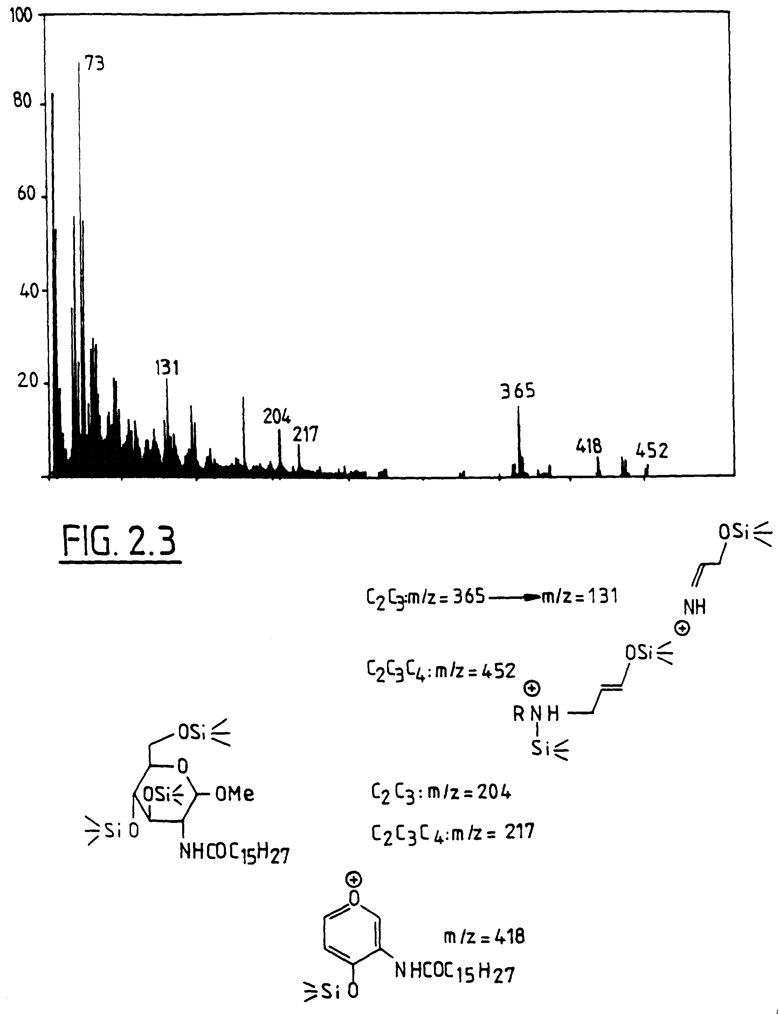

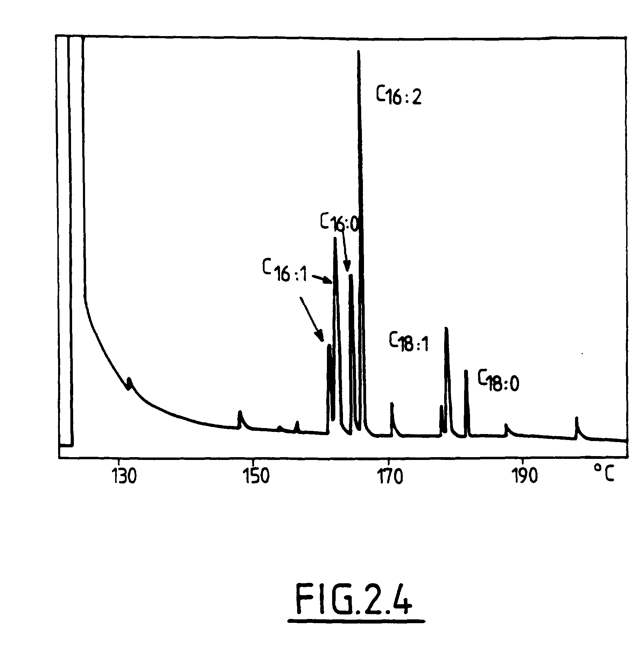

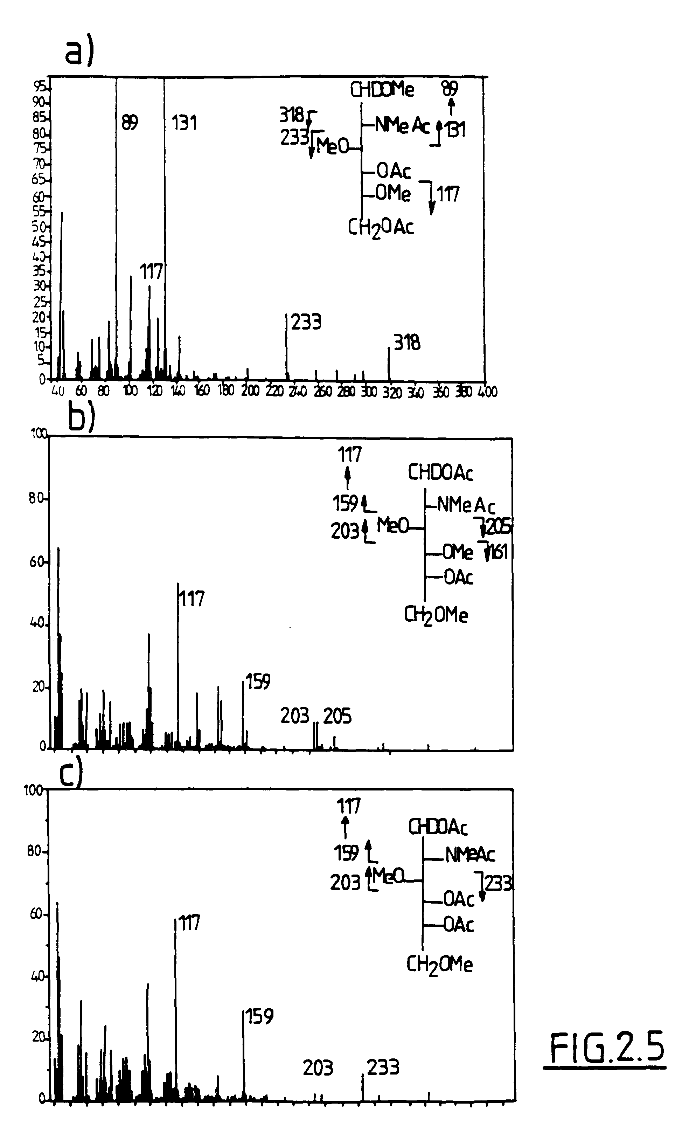

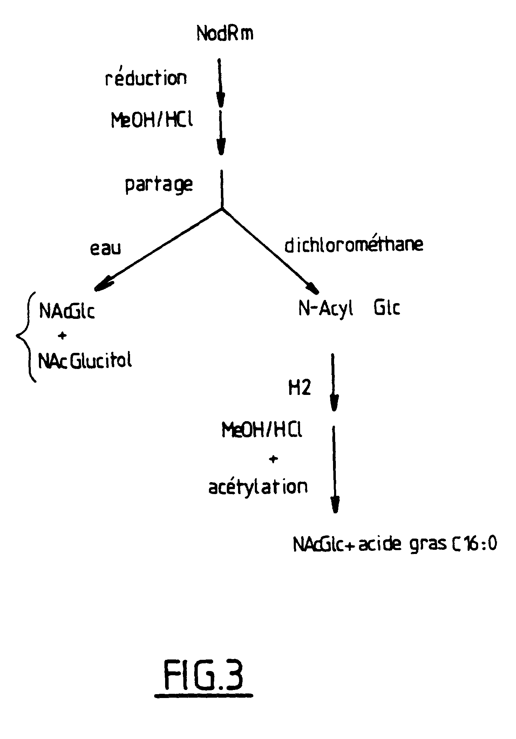

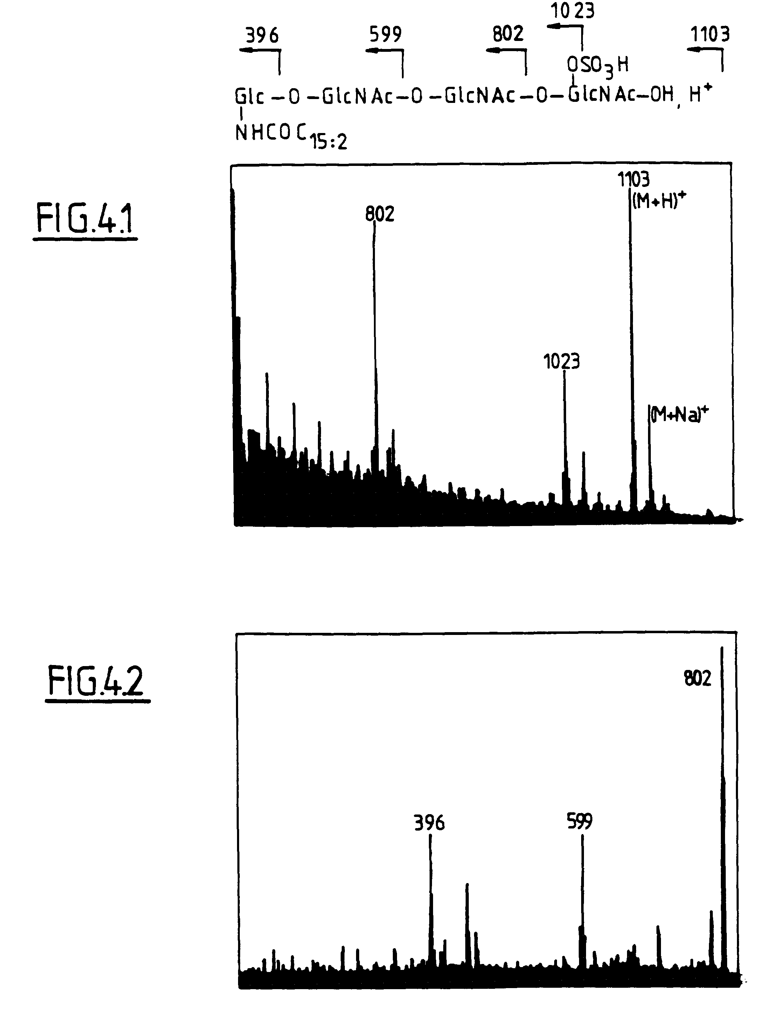

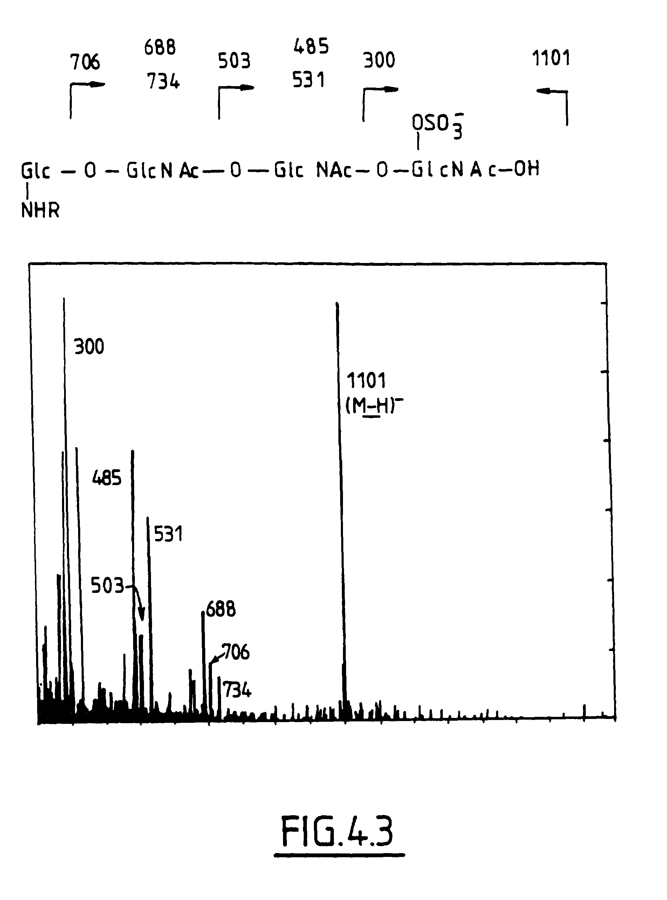

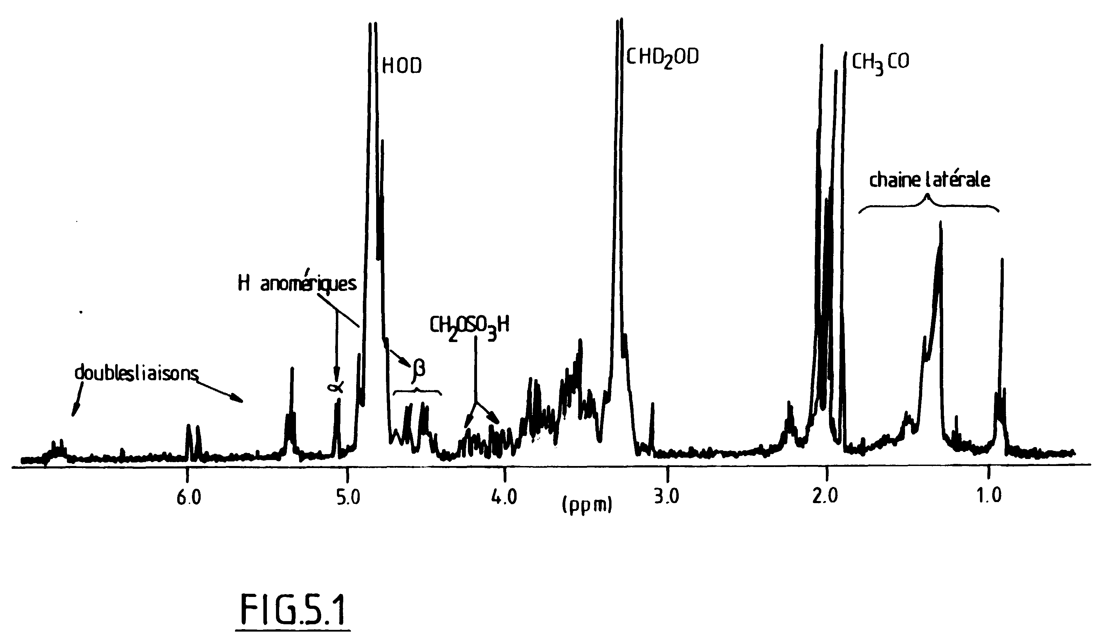

**B.2.1. Analyse des monosaccharides**
   Après hydrolyse poussée du composé (HCl 3N; 3h; 100°C), puis dérivation en glycoside de méthyle péracétylé, on observe en chromatographie en phase vapeur (CPV) un seul monosaccharide identifié comme étant du déoxy-2-acétamido-2 glucose ou de l'acétyl glucosamine (NAc Glc). La série D de ce sucre a été établie après préparation du glycoside peracétate avec un alcool chiral et comparaison en CPV avec des témoins de (-) butanol-2-N-acétylglucosamine des séries D et L dérivés de façon analogue.
   La réduction du composé au borodeuterure de sodium (NaBD₄/H₂0 1N;18h; 20°C) est suivie d'une méthanolyse douce (MeOH/HCI 1N; 80°C; 1h) puis d'un partage eau-dichlorométhane. La phase aqueuse contient du GlnNac-1-0-Me et du deoxy-2-acétamido-2-glucitol, identifiés en CPV (Fig. 2.1) ; l'identification en CPV est réalisée sur une colonne OV1 0,32 mmX30m, en utilisant l'hélium et un détecteur FID. La phase organique renferme le méthylglycoside de la glucosamine N-acylée par un acide gras C_{16:2} identifiée par son spectre de masse en impact électronique du dérivé pertriméthylsilylé selon les données de la littérature (Demary, M. et al., Nouv. J. Chimie, 1977,2,373-378) ; le spectre de masse impact électronique (EI-MS) de la glucosamine 1-OMe amidifiée sur l'atome d'azote par un acide gras et pertriméthylsilylée est représenté à la figure 2.3. Après hydrogénation catalytique de la chaîne latérale, méthanolyse (MeOH, HCl 3N; 2h; 100°C), puis acétylation, l'analyse en CPV - réalisée sur colonne OV1 0,32mmX30m à l'aide d'hélium et d'un détecteur FID - des dérivés peracétylés permet de confirmer la structure de ce N-acyl-sucre en visualisant du Glc-NAc-1-OMe et du palmitate de méthyle (accompagné de stéarate de méthyle en quantité moindre) (Fig. 2.2).
   Cette analyse permet d'établir la présence de trois types de glucosamine: NAcGlc, NacGlc réductrice et N-acyl Glc, selon un cheminement analytique résumé à la Fig. 3 annexée.
**B.2.2. Composition en acides gras**
   Les acides gras présents dans le composé ont pu être isolés après saponification (KOH 5%;18h; 80°C) puis analysés en CPV - sur colonne OV1 0,32mmX30m à l'aide d'hélium et d'un détecteur FID - sous la forme d'esters de méthyle (Fig. 2.4). Cette étude a permis d'identifier outre un acide gras majoritaire en C_{16:2}, d'autres entités minoritaires: C_{16:0}, C_{16:1}, C_{18:0} et C_{18:1}. La position des deux double-liaisons de l'acide gras en C_{16:2} a pu être précisée à partir du spectre de masse MS-MS du carboxylate formé à partir de son ester de perfluorobenzyle (J.C. Promé et al., Rapid Comm. Mass spectrom., 1987,1,80-82) et localisée en 2 et 9 sur la chaîne.
**B.2.3. Mise en évidence d'une fonction sulfate**
   Après culture des bactéries en présence de sulfate de sodium marqué au ³⁵S, puis purification du composé, la correspondance observée entre le profil d'absorption UV et l'incorporation de ³⁵S permet de conclure à la présence d'une fonction sulfate dans ce composé. La figure 1.4b représente ladite correspondance obtenue par incorporation de ³⁵S, après culture sur (³⁵S) Na₂SO₄ puis fractionnement selon le profil représenté à la figure 1.4a et comptage de la radioactivité par scintillation liquide.
**B.2.4. Détermination du mode de liaison interglycosidique**
   Le mode de liaison entre les différentes glucosamines a été établi après perméthylation du composé réduit par NaBD₄, hydrolyse et préparation des alditols acétates selon les méthodes précédemment décrites (K. Stellner et al., Arch. Biochem. Biophys., 1973,155,464-472), suivies d'une analyse par CPV-SM ; le spectre de masse impact électronique des alditols acétates issus de la perméthylation du composé réduit est représenté à la figure 2.5. Cette étude a permis d'identifier des alditols acétates tetra-méthylés en 1,2,3,5 (spectre a) et 2,3,4,6 (spectre b) et tri-O-méthylés 2,3,6 (spectre c) provenant respectivement d'une glucosamine réductrice substituée en 4 et 6, d'une glucosamine terminale et d'une glucosamine liée en 1 et 4.

### B.3. Spectrométrie de masse

**B.3.1. Spectrométrie de masse FAB mode positif**
   Le spectre de masse FAB mode positif du composé (Fig. 4.1, balayage de m/z 4 000 à m/z 100 (masse nominale)) montre un ion pseudomoléculaire M+H⁺ m/z=1103, accompagné de l'ion sodé correspondant à m/z=1125. Des ions fragmentaires sont aussi observés à m/z=1023 et 802 attribués respectivement aux pertes de sulfate et de NAcGlucosamine sulfate. La décomposition de l'ion m/z=802 est observée en spectrométrie MS-MS (Fig. 4.2; balayage en B/E) et conduit à deux fragments glycosilyle à m/z=599 et 396 consécutifs aux cassures entre les différents monosaccharides. Ces deux spectres permettent d'établir une structure tétrasaccharidique linéaire dont le sucre réducteur est substitué par une fonction sulfate et le sucre terminal par un acide gras en C_{16:2}.
**B.3.2. Spectrométrie de masse FAB mode négatif**
   Hormis l'ion pseudomoléculaire M-H⁻ à m/z=1101, le spectre FAB mode négatif présente des fragments attribuables aux coupures glycosidiques. L'analyse de ces ions conduit à des conclusions structurales analogues à celles décrites plus haut : cf. la figure 4.3 qui montre le spectre de masse d'ionisation FAB négatif, en utilisant le thioglycérol comme matrice.

### B.4. RMN

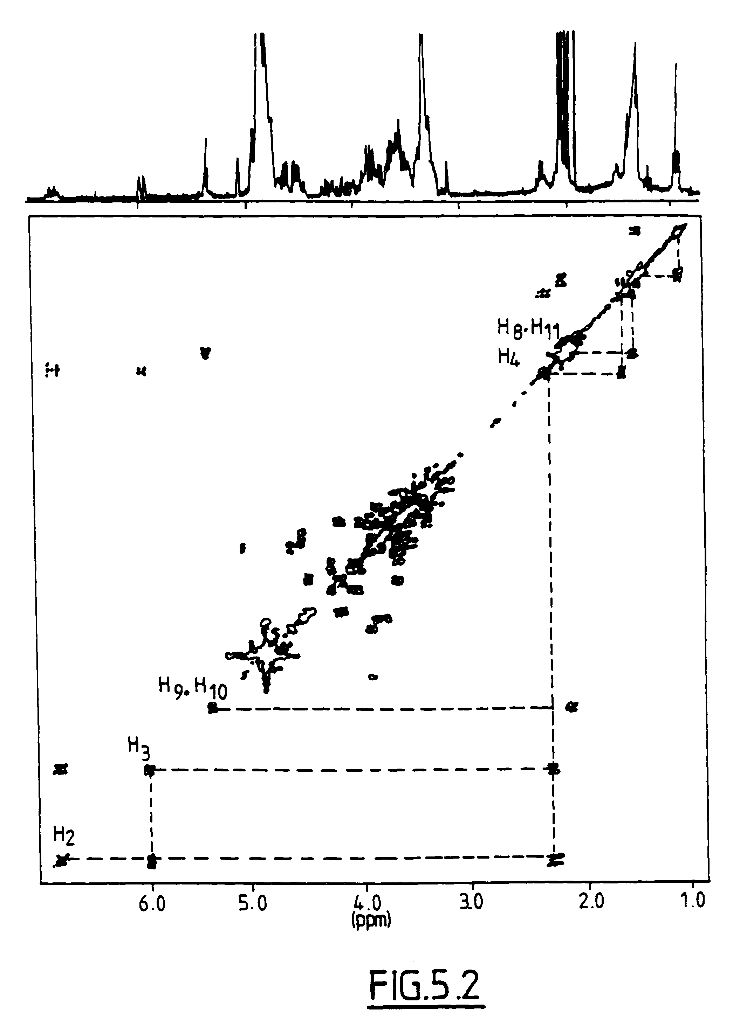

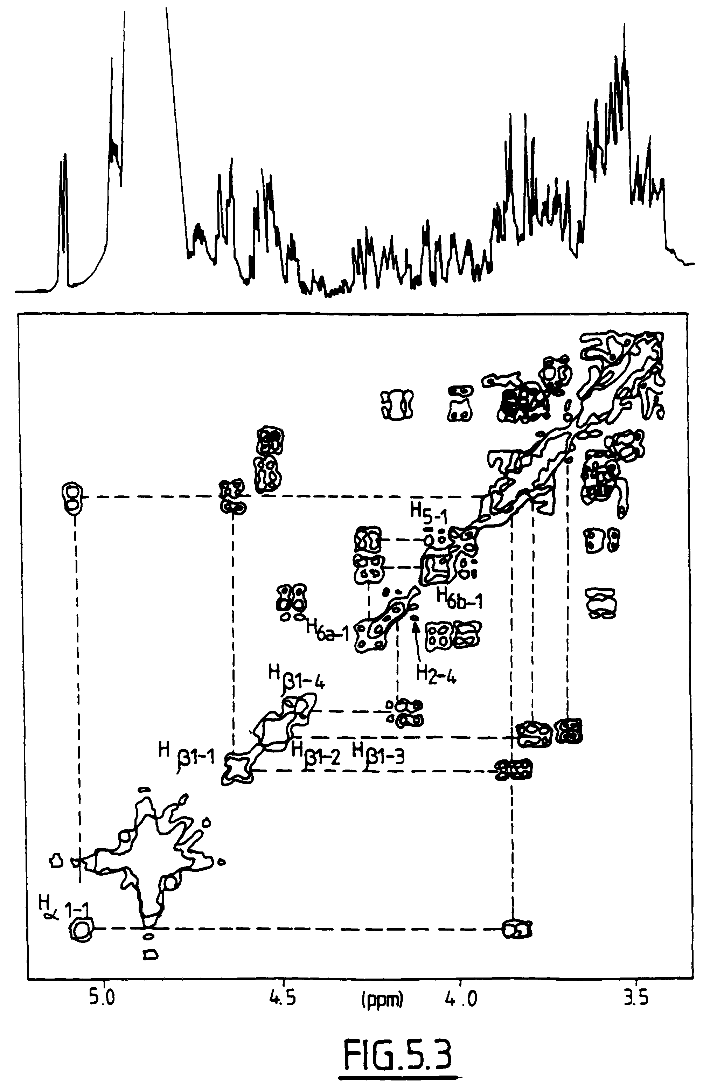

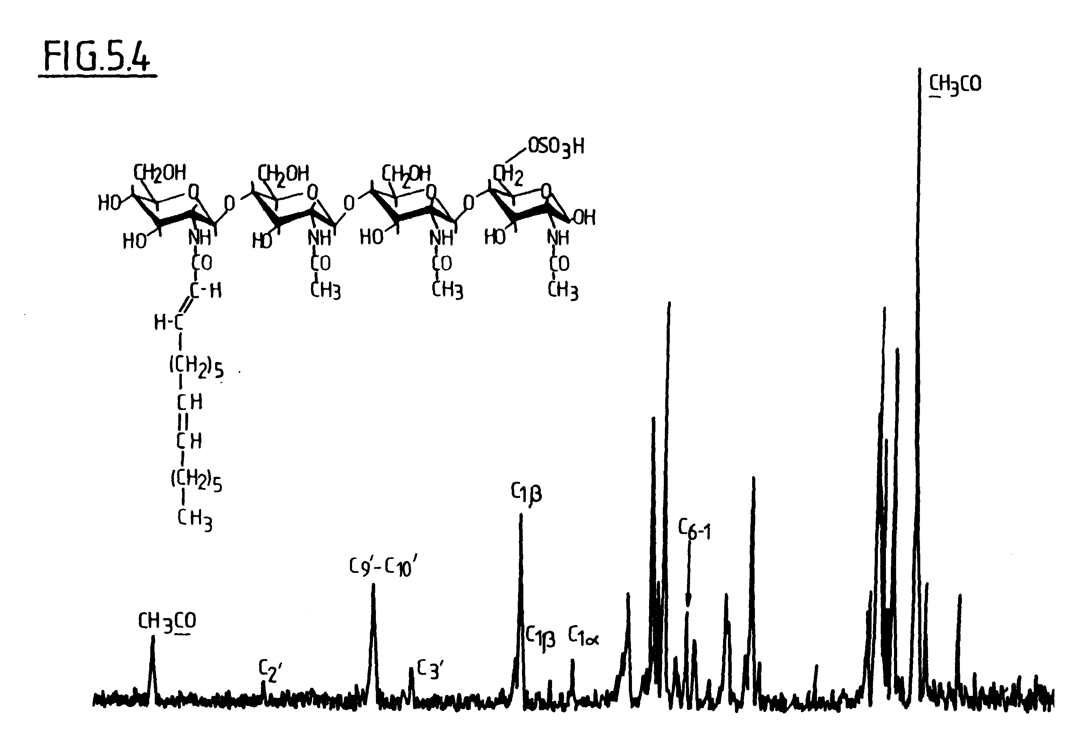

**B.4.1. RMN**^{**1**}**H et RMN-2D-homonucléaire proton (COSY)**
   Les données relatives aux spectres RMN¹H et RMN-COSY permettent d'établir les éléments structuraux suivants :
   a) La chaîne latérale présente en RMN¹H deux types de protons vinyliques correspondant aux deux double-liaisons. Deux signaux à bas champ (δ=5, 95ppm;1H et δ=6,80ppm;1H) sont attribués à une double liaison conjuguée à la fonction carbonyle et présentent une grande constante de couplage (J=15Hz) caractéristique d'une géométrie E ; la figure 5.1 représente le spectre RMN¹H susdit ; on utilise comme solvant le CD₃OD et une fréquence de 300MHz (Appareil "Bruker"). Les deux protons de la double-liai-son interne à la chaîne, magnétiquement équivalents, apparaissent en un seul signal (δ=5,35ppm). Les protons de ces deux double-liaisons présentent en 2D-COSY une cascade de corrélations avec les autres protons aliphatiques caractéristique d'une chaîne linéaire ; la figure 5.2 représente le spectre RMN-2D-COSY homonucléaire¹H obtenu en utilisant le CD₃OD comme solvant et une fréquence de 300MHz (Appareil "Bruker").
   b) Protons anomériques : Le spectre RMN¹H-2D-COSY homonucléaire de la région saccharidique est représenté à la figure 5.3 ; ce spectre qui a été obtenu en utilisant comme solvant le CD₃OD et une fréquence de 300MHz (Appareil "Bruker") montre dans la région des protons saccharidiques de 3,4 à 5,2 ppm 5 signaux n'ayant qu'une corrélation, attribués aux protons anomériques. De plus, aucun signal n'est masqué par le massif de l'eau comme l'indique l'absence de corrélation de cette région du spectre. Ces signaux anomériques correspondent à trois doublets de liaisons β (δ=4,40 à 4,55ppm; 3H; J=8,5Hz) et deux doublets attribués aux protons anomériques des formes α et β du sucre réducteur (δ(α)=5,05ppm;J=3,4Hz et δ(β)=4, 60ppm; J=8,5Hz).
   c) Protons des cycles : Les cycles saccharidiques sont caractérisés par les signaux méthyle des groupements acétamide, dédoublés par la coexistence des formes α et β libres de l'oligosaccharide (δ=2,0 à 2,15ppm; 6s; 9H). Les autres protons du cycle résonnent entre 3,2 et 3,9 ppm. Seuls trois signaux plus déblindés sont attribués aux hydrogènes situés à proximité du groupement sulfate. Le mode de corrélation observé en 2D-COSY (Fig. 5.3) entre ces trois signaux est caractéristique de protons 5 et 6 (corrélation H5/H6b, H5/H6a et H6a/H6b). Ce résultat permet de situer la fonction sulfate en position 6.
**B.4.2. RMN**^{**13**}**C**
   L'attribution des différents signaux du spectre RMN¹³C découplé du proton (Fig. 5.4), a permis de confirmer la présence d'une chaîne d'acide gras di-insaturé, le mode de liaison β 1→4 entre les glucosamines ainsi que la position en 6 du sulfate à partir des éléments suivants ; La figure 5.4 représente le spectre RMN¹³C obtenu à 50,4MHz (Appareil "Bruker"), en utilisant comme solvant le D₂O:
   a) Chaîne d'acide gras : Les déplacements chimiques des carbones de la chaîne latérale (C_{2'} à C_{16'}) sont caractéristiques d'un acide gras possédant une double-liaison conjuguée (C_{2'} et C_{3'};δ=125 et 155 ppm) et une double liaison interne (C_{9'} et C_{10'};δ=133 ppm).
   b) Carbones anomériques : Un signal prépondérant (δ=103,7ppm) attribué aux carbones anomériques des monosaccharides non réducteurs, présente un déplacement chimique analogue au C₁ du glucopyranoside de méthyle du GlcNAc. Deux signaux d'intensités plus faibles sont attribués au C₁ de la glucosamine réductrice apparaissant sous ces deux formes anomériques libres (δC₁α=93,1ppm et δc₁β=97,8ppm).
   c) Cycle : A partir des données de la littérature, l'attribution des différents signaux conduit à confirmer l'enchaînement β 1→4 dans le tétrasaccharide. Cette attribution est compliquée par la séparation en deux groupes de signaux des carbones de la glucosamine réductrice. La présence de la fonction sulfate en 6 est confortée par le déblindage observé du carbone 6 porteur de cette fonction (δC₆₋₁=68,7ppm).

### Exemple 2 - Mise en évidence de l'activité biologique du facteur NodRm-1

Les activités biologiques mises en évidence (i) par le test d'activité spécifique de déformation des poils racinaires (Had) sur la vesce décrit par Zaat et al., (J. Bactériol., 1987,169,3388-3391), (ii) et par les tests de déformation et de ramification des poils racinaires de luzerne décrits par Faucher et al. (J. Bactériol, 1988,170,5489-5499 ; Molec. Plant-Microbe Interact., 1989,2,291-300) ont prouvé que la substance conforme à l'Invention est un signal symbiotique plante-spécifique dont la production est sous le contrôle des gènes *nod.*

En effet :
1) après trois types de purifications basés sur des propriétés physico-chimiques différentes, échange d'ions, filtration en gel et la chromatographie en phase inverse, on observe dans tous les cas une corrélation absolue entre la présence de cette molécule - caractérisée par son profil HPLC, par spectrométrie de masse et par spectrométrie RNM-¹H- et son activité Had spécifique sur la luzerne ;
2) une augmentation de l'activité des gènes *nod* soit par induction de la transcription, soit par l'augmentation du nombre de copies de ces gènes, est corrélée avec une augmentation de la production de la molécule, alors qu'une mutation due au transposon Tn5 dans les gènes *nodA* ou *nodC* a pour effet de supprimer sa production ;
3) L'activité biologique du composé NodRm-1, telle que détectée par les bio-essais de ramification des poils racinaires est très élevée et spécifique.

Une solution NodRm-1 à une concentration de l'ordre de 10⁻⁸-10⁻¹⁰ M provoque des réactions Had sur la luzerne, hôte homologue, mais pas sur la vesce, qui est un hôte hétérologue. Le composé NodRm-1 (voir formule II) est encore actif vis-à-vis de la luzerne, à une concentration de 10⁻¹¹ M.

Le spectre RNM¹H indique pour le composé NodRm-1 une pureté d'au moins 95 % (cf. Fig. 5.1).

Il n'a pas été détecté, dans la fraction active, de molécules comportant un noyau aromatique caractéristique des hormones végétales telles que l'auxine et la cytokinine. C'est pourquoi les contaminants hormonaux éventuels devraient être présents à des concentrations inférieures à 10⁻¹² M, c'est-à-dire à des concentrations environ 100 000 fois inférieures au seuil (10⁻⁷ M) auquel ces phythormones agissent lorsqu'elles sont ajoutées par voie exogène, ce qui signifie que l'on ne peut pas attribuer à de tels contaminants les effets observés.

L'étude des mutants de *R.meliloti* affectés dans des gènes hôtes-spécifiques fait apparaître une corrélation absolue entre la spécificité du comportement symbiotique de cellules vivantes de *Rhizobium* et la spécificité du bio-essai Had de leurs surnageants stériles, ce qui indique que les signaux Nod sont impliqués dans l'induction de l'infection spécifique et de la nodulation.

Le composé NodRm-1 a à la fois une très forte activité biologique et une très forte spécificité. En effet, il provoque des réactions détectables sur la plante-hôte à des concentrations très faibles, de l'ordre de 10⁻¹² M, alors que les hormones végétales connues, comme les auxines et cytokinines, agissent à des concentrations nettement supérieures (10⁻⁷-10⁻⁸ M). D'autre part, les oligosaccharides de type "éliciteurs" qui ont été décrits dans l'Art Antérieur et qui agissent à des concentrations faibles (10⁻⁹ M) ne sont pas spécifiques, alors que la molécule-signal NodRm-1 conforme à l'Invention a une grande spécificité d'action : alors qu'elle agit à une concentration de 10⁻¹² M sur la luzerne, plante homologue, elle n'est toujours pas active à une concentration de 10⁻⁸ M sur une plante non-hôte comme la vesce. Cette molécule présente donc des propriétés originales et importantes quant à son activité sur les plantes.

En l'absence de bactéries et à de faible concentrations (10⁻⁷ M-10⁻⁹ M) la molécule-signal NodRm-1 provoque l'induction d'abondantes divisions mitotiques dans le cortex racinaire de la luzerne : NodRm-1 présente donc un fort effet mitogène.

En l'absence de bactéries et à de faible concentrations (10⁻⁷ M-10⁻⁹ M) la molécule-signal NodRm-1 provoque l'induction de la formation de nodosités sur les racines de la luzerne, nodosités qui présentent des caractéristiques ontologiques, morphologiques et anatomiques des nodosités de luzerne induites par les bactéries symbiotiques. NodRm-1 a donc un effet organogène sur les racines de luzerne.

En l'absence de bactéries et à de très faibles concentrations (10⁻⁹ M 10⁻¹² M), le composé NodRm-1 provoque des déformations de poils absorbants sur la luzerne et une induction de la transcription de gènes symbiotiques de Légumineuses. A ces mêmes doses en présence de *R.meliloti,* l'addition de composé NodRm-1 provoque une accélération de l'infection et de la formation des nodosités racinaires. Son addition à des Légumineuses, par exemple par enrobage de graines au moment du semis, est apte à accélérer la formation des nodosités et l'établissement de la fixation d'azote.

Il est important de souligner que c'est la première fois qu'on isole à partir d'une bactérie des oligomères d'hexosamines sulfatés. En effet, des sucres sulfatés ont été isolés chez l'animal mais n'avaient été ni détectés ni a fortiori isolés jusqu'à ce jour ni chez les plantes ni chez les bactéries.

De plus, les oligomères d'hexosamines sulfatés et acylés conformes à la présente Invention présentent une activité biologique exceptionnelle en ce qu'ils ont une activité à des doses infinitésimales ; en effet, ils sont au moins 100 000 fois plus actifs que les hormones végétales. On n'a jamais proposé jusqu'à présent l'utilisation de substances naturelles ayant une telle activité à des doses aussi faibles, dans le domaine végétal. Cette activité exceptionnelle laisse à penser que les substances conformes à l'Invention ont une affinité considérable pour certains récepteurs - affinité très supérieure à celle des produits décrits jusqu'à présent y compris les hormones végétales -. Les récepteurs qui ont une affinité pour les sucres présentent généralement la structure de glycoprotéines et notamment de lectines ; or de tels récepteurs existent aussi dans le règne animal ; les substances conformes à l'Invention doivent donc être considérées comme pouvant être dotées d'une activité thérapeutique importante. En particulier, l'agent thérapeutique conforme à l'Invention est propre, entre autres, à stimuler les mécanismes de défense contre les pathogènes chez l'Homme et chez l'animal.

### Exemple 3 - Préparation des substances Ac-NodRm-1 et Ac-NodRM-3 par purification à partir d'un milieu de culture de Rhizobium meliloti

### A. Production des composés

Si au lieu d'introduire le plasmide "surproducteur" pGM1149 dans la souche de *R. meliloti* 2011, on introduit le plasmide pMH682 qui contient seulement le couple de gènes régulateurs *syrM/NodD3,* on provoque également une forte augmentation de la transcription des gènes *nod* communs et spécifiques et une augmentation de la production de facteurs Nod. Les extraits butanoliques du surnageant d'une telle souche, lorsqu'ils sont fractionnés par chromatographie HPLC, présentent un profil différent de celui des extraits de la souche *R. meliloti* 2011 (pGM1149), ce qui implique la production de nouveaux composés.

### B. Détermination de la structure des composés

### 1. Purification

Les composés sont purifiés selon le protocole décrit dans l'exemple 1. L'ultime étape de chromatrographie HPLC analytique en phase inverse permet de séparer le composé Ac-NodRMm-1 du composé Ac-NodRm-3.

### 2. Etudes structurales

### 2.1. Structure du composé AC-NodRm-1

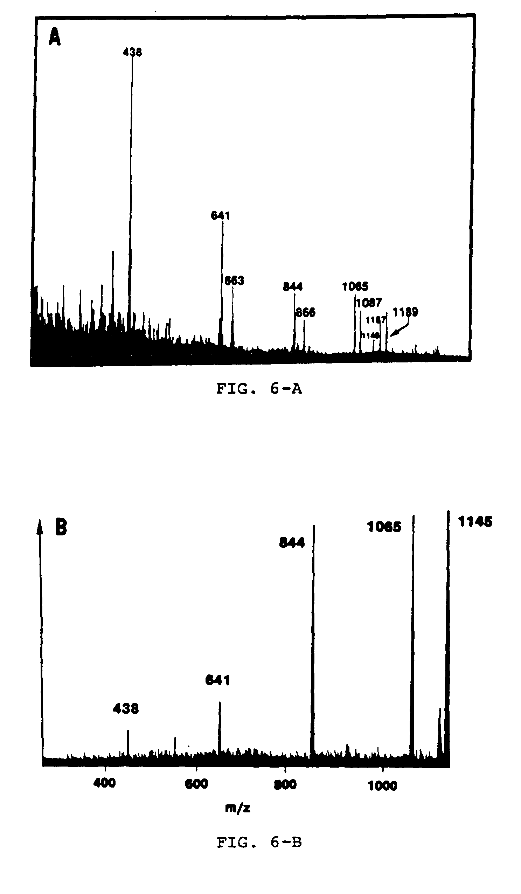

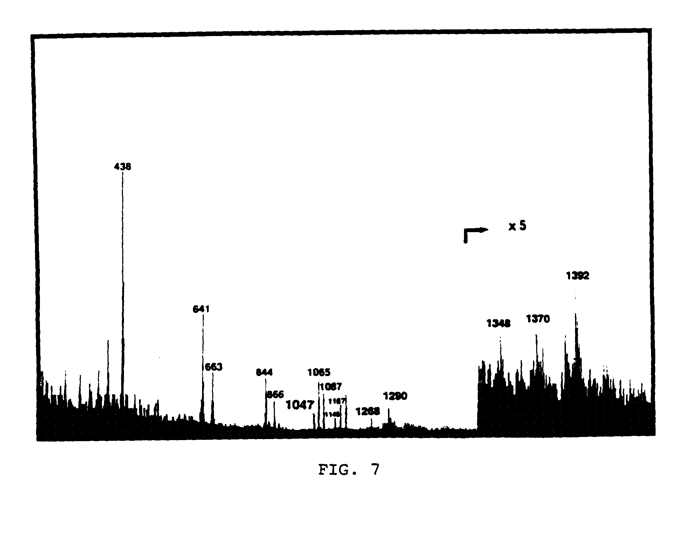

2.1.1. Traitement au méthanolate de sodium
   Par traitement au methanolate de sodium, le composé Ac-NodRm-1 est converti en NodRm-1 dont la structure est déterminée comme dans l'exemple 1.
2.1.2. Spectrométrie de masse
   Le spectre de masse FAB mode positif du composé Ac-NodRm-1 (Fig 6A) montre un ion moléculaire protonné à m/z 1145, ainsi que les ions (M+Na)⁺ à m/z 1167 et (M+2Nₐ-H)⁺ à m/z = 1189. La décomposition de l'ion moléculaire protonné par spectrométrie MS/MS (Fig. 6B : balayage B/E) conduit à des ions fragmentaires à m/z 1065, m/z 844, m/z 641 et m/z 438. Les ions fragments à m/z 1065 et m/z 844 sont attribués respectivement à la perte de SO₃ et de N-acétyl-D-glucosamine sulfate. Les fragments à m/z 641 et m/z 438 correspondent aux ions glycosylium obtenus par rupture des liaisons interglycosidiques. Ces deux spectres montrent que le composé Ac-NodRm-1 ne diffère du composé NodRm-1 que par la présence d'un groupement acétate sur la glucosamine terminale non réductrice.
2.1.3. RMN¹H
   Le spectre RMN¹H du composé Ac-NodRm-1 permet d'établir les éléments structuraux décrits dans l'exemple 1 (paragraphe B.4.1.) pour le composé NodRm-1.
   (i) deux doubles liaisons dont une conjuguée de géométrie E ;
   (ii) signaux anomériques caractéristiques de liaison β
   (iii) signaux caractéristiques d'un groupe sulfate en position 6.

De plus, deux signaux à δ = 4,16 ppm et δ = 4,46 ppm, absents dans le spectre du composé NodRm-1 et disparaissant après O-déacétylation, permettent de localiser le groupement acétate en position 6 du sucre terminal non réducteur

### 2.2. Structure du composé Ac-NodRm-3

La structure du composé Ac-NodRm-3 a été établie par spectrométrie de masse FAB mode positif (Fig.). On observe un ion moléculaire protonné à m/z = 1348 ainsi que des ions sodés (M+Na)⁺ à m/z = 1370 et (M+2Na-H)⁺ à m/z = 1392. Les ions à m/z = 1268 et m/z = 1047 sont attribués respectivement à la perte de SO3 et d'une N-acétyl-glucosamine sulfate à partir de (M+H)⁺. Les ions à m/z = 844, m/z = 641 et m/z = 438 sont attribués aux ions glycosylium obtenus par coupures interglycosidiques. Ce spectre indique qu'Ac-NodRm-3 :
(i) est constitué d'un enchaînement linéaire de cinq dérivés de D-glucosamine ;
(ii) possède une chaîne N-acyl diinsaturée de 16 carbones et un groupement O-acétate sur le résidu glucosamine terminal non réducteur ;
(iii) possède une fonction sulfate sur le résidu N-acétyl-D-glucosamine réducteur.

L'incubation du composé Ac-NodRm-3 en présence d'une exochitinase (extraite de *Streptococcus griseus,* Sigma) libère notamment un dimère de N-acétyl-D-glucosamine et de N-acyl-O-acétyl-D-glucosamine, ce qui montre que les liaisons entre les trois premiers résidus N-acétyl-D-glucosamine sont de type β-1,4. La digestion d'Ac-Nod3m-3 par une endochitinase (également extraite de *Streptococcus griseus,* Boeringer) conduit à la formation d'une N-acyl-O-acétyl-D-glucosamine montrant que la liaison avec le résidu terminal non réducteur est également de type β-1,4.

### Exemple 4 - Préparation de facteurs Nod à partir d'un milieu de culture de Rhizobium leguminosarum biovar viciae

### A. Production des facteurs Nod

Dans les pays européens la culture de protéagineux, comme le Pois et le Féverole, s'est beaucoup développée au cours de la dernière décennie. Les Inventeurs ont donc entrepris d'étudier la production de facteur Nod par *Rhizobium leguminosarum* b.v. *viciae*, la bactérie symbiotique de ces protéagineux.

Dans le but de provoquer une augmentation de la production de ces signaux, les Inventeurs ont introduit dans la souche *R*. *leguminosarum* 248, le plasmide plJ1089. Ce plasmide, dérivé du vecteur pLAFR1, contient l'ensemble de la région Nod de *R. leguminosarum,* c'est-à-dire les opérons *nodD, nodABCIJ*, *nodFEL*, *nodMNT, nodO* (Downie et al., EMBO J., 1983, 2, 947-952).

La souche 248(plJ1089) est cultivée dans le même milieu que *R. meliloti*, mais pour l'induction de la transcription des gènes *nod,* une flavanone, la naringénine, a été utilisée à la place de la lutéoline. Après induction des gènes *nod* le surnageant des cultures présente une forte activité de déformation de poils absorbants d'une espèce de vesce appropriée *(Vicia sativa* subsp. *nigra*)*.* Cette activité n'est pas détectable dans les surnageants de culture d'une souche *R. leguminosarum* 248 mutée dans les gènes communs *nodABC*.

### B. Purification

Les composés Nod sont extraits du milieu de culture selon le procédé décrit dans l'exemple 1 (B,1.1.). L'extrait est lyophilisé puis chromatrographié en HPLC phase inverse selon le procédé décrit dans l'exemple 1 (B.1.2.). Les composés Nod sont ensuite purifiés sur une cartouche SEP-PAK® QMA (Waters Millipore) forme acétate. Les composés sont élués avec 5 ml d'éthanol absolu.

### C. Détermination de la structure

Le spectre de masse FAB en mode positif montre majoritairement trois ions moléculaires protonnés à m/z = 1067, m/z = 1069 et m/z = 1095, accompagnés de leurs ions sodés (M+Na)⁺ respectivement à m/z = 1089, m/z = 1091 et m/z = 1117. La décomposition par spectrométrie MS/MS de l'ion à m/z = 1089 (respectivement m/z = 1091 et m/z = 1117) fournit des ions fragments à m/z = 868 (respectivement m/z = 870 et m/z = 896), m/z = 665 (respectivement m/z = 667 et m/z = 693) et m/z = 462 (respectivement m/z = 464 et m/z = 490). Ces ions sont obtenus par rupture interglycosidique, avec perte successive de fragments d'une masse de 203 correspondant à un résidu N-acétyl-glucosamine. Ces spectres montrent que les composés NodRl sont constitués d'un enchainement linéaire de N-acétyl-hexosamine. Les ions de m/z = 462, 464 et 490 correspondent à des O-acétyl-hexosamines diversement N-acylées.

L'analyse des acides gras portés par le résidu terminal non-réducteur a été réalisée par GC/MS après saponification selon le protocole décrit dans le paragraphe B.2.2. Cette étude a permis d'identifier les acides gras majoritaires suivants : C_{16:0}, C_{15:1} et C_{18:1}.

## Revendications

1. Utilisation d'un lipo-oligosaccharide ou d'un mélange de lipo-oligosaccharides comprenant un oligomère d'hexosamine, dont l'hexosamine terminale non réductrice est N-substituée par un acide gras, en tant que signal symbiotique, plante-spécifique pour favoriser la symbiose Rhizobiacées-légumineuses.

2. Utilisation selon la Revendication 1, caractérisée en ce que la glucosamine terminale non-réductrice de l'oligomère de N-acétyl glucosamine est N-substituée par un acide gras comprenant au moins 12 atomes de carbone.

3. Lipo-oligosaccharide caractérisé en ce qu'il répond à la formule générale I ci-après : 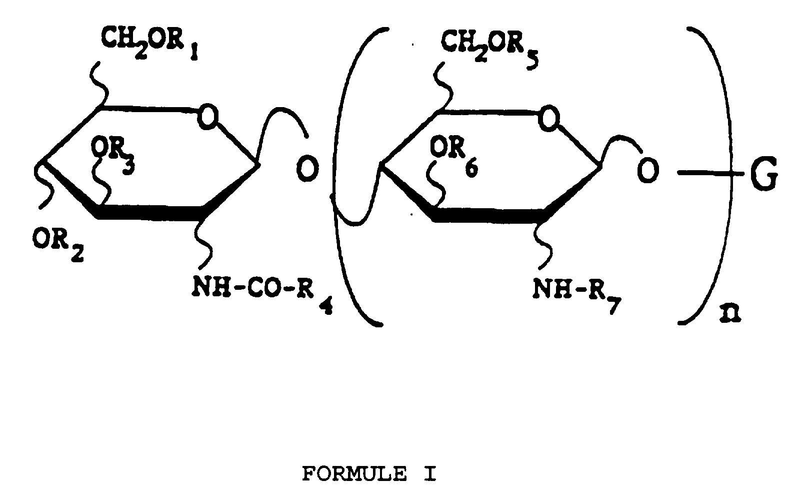 dans laquelle :
- G est une hexosamine diversement substituée, par exemple, par un groupe acétyl sur l'azote, un groupe sulfate, un groupe acétyl et/ou un groupe éther sur un oxygène.
- R1, R2, R3, R5, R6, R7, qui peuvent être identiques ou différents, représentent H, CH3CO-, CXHYCO-, où x est un nombre entier compris entre 0 et 17 et y est un nombre entier compris entre 1 et 35, ou tout autre groupe acyl, tel que par exemple un carbamyl
- R4 représente une chaîne aliphatique saturée ou mono-, di-, ou tri-insaturée comportant au moins 12 atomes de carbone, et
- n est un nombre entier compris entre 1 et 4.

4. Lipo-oligosaccharide selon la Revendication 3, caractérisé en ce que G représente :
. Chez *R. meliloti,* une N-acétyl-D-glucosamine-6-sulfate
. Chez R. *leguminosarum b.v. viciae,* une N-acétyl-D-gluco samine

5. Lipo-oligosaccharide selon l'une quelconque des Revendications 3 ou 4, caractérisé en ce qu'il répond à la formule (II) ci-après : 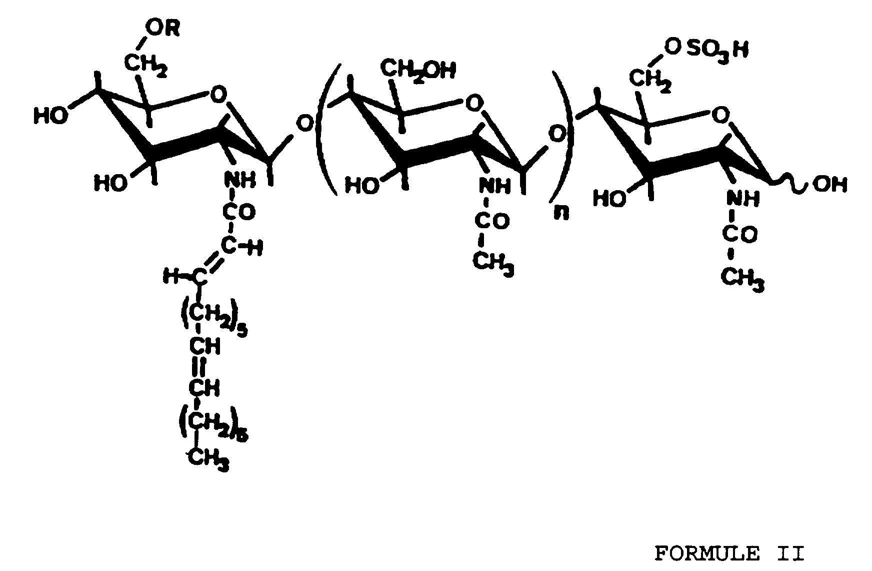 dans laquelle R représente H ou CH₃CO- et n est égal à 2 ou 3.

6. Procédé de production d'un lipo-oligosaccharide selon l'une quelconque des Revendications 3 à 5, par purification, caractérisé en ce qu'au cours d'une première étape on produit un plasmide recombinant résultant du clonage dans un plasmide capable de se répliquer dans une bactérie Rhizobiacée ou toute autre bactérie appropriée, (1) soit d'un fragment qui contient des gènes *nod* communs et spécifiques ainsi que les gènes régulateurs d'une bactérie Rhizobiacée donnée, soit (2) des gènes régulateurs de l'expression des gènes *nod,* les bactéries Rhizobiacées ou toute autre bactérie appropriée sont ensuite modifiées par introduction dudit plasmide recombinant, pour obtenir une souche mutante très surproductrice de facteurs Nod, et on cultive ladite souche mutante dans un milieu de culture approprié ; - au cours d'une deuxième étape on recueille le surnageant de culture, que l'on purifie par extraction par un alcool inférieur approprié, ou par extraction solide-liquide appropriée, suivie d'une chromatographie HPLC en phase inverse du résidu d'extraction, d'une chromatographie par perméation de gel, d'une chromatographie par échange d'ions et d'une chromatographie HPLC analytique en phase inverse.

7. Procédé selon la Revendication 6, caractérisé en ce que le plasmide surproducteur de facteur Nod est introduit dans une bactérie Rhizobiacée mutante non-productrice d'exopolysaccharides.

8. Procédé de production de lipo-oligosaccharides selon l'une quelconque des Revendications 3 à 5, caractérisé en ce qu'on part d'une souche sauvage de bactéries Rhizobiacées fortement productrice de facteurs Nod, que l'on cultive dans un milieu de culture approprié, après quoi l'on recueille le surnageant de culture que l'on purifie conformément à la 2ème étape du procédé selon la Revendication 6.

9. Agent de traitement de plantes caractérisé en ce que son ou un constituant actif est un lipo-oligosaccharide selon l'une quelconque des Revendications 3 à 5.

10. Agent de traitement de plantes selon la Revendication 9, caractérisé en ce que ledit lipo-oligosaccharide est présent à une concentration comprise entre 10⁻⁶ M et 10⁻¹⁴ M.

## Patentansprüche

1. Verwendung eines Lipo-Oligosaccharids oder eines Gemisches von Lipo-Oligosacchariden, das bzw. die ein Hexosamin-Oligomeres umfaßt bzw. umfassen, dessen nichtreduzierende Hexosamin-Endgruppe durch eine Fettsäure N-substituiert ist, als pflanzenspezifisches symbiotisches Signal zur Begünstigung der Symbiose zwischen Knöllchenbakterien (Rhizobiaceae) und Leguminosen.

2. Verwendung nach Anspruch 1, dadurch **gekennzeichnet**, daß die nichtreduzierende Glucosamin-Endgruppe des N-Acetylglucosamin-Oligomeren durch eine Fettsäure mit mindestens 12 Kohlenstoffatomen N-substituiert ist.

3. Lipo-Oligosaccharid, dadurch **gekennzeichnet**, daß es der folgenden allgemeinen Formel I entspricht: 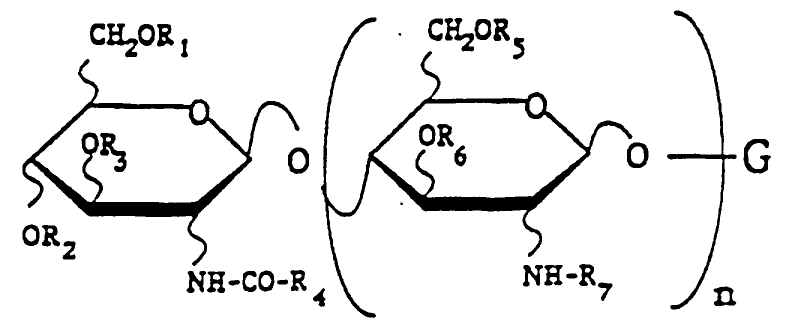
worin G für ein Hexosamin steht, das verschieden substituiert ist, z.B. durch eine Acetylgruppe am Stickstoff, eine Sulfatgruppe, eine Acetylgruppe und/oder eine Ethergruppe an einem Sauerstoff,
R₁, R₂, R₃, R₅, R₆, R₇, die gleich oder verschieden sein können, für H, CH₃CO-, CXHYCO-, worin x eine ganze Zahl von 0 bis 17 und y eine ganze Zahl von 1 bis 35 ist, oder eine beliebige Acylgruppe, wie z.B. eine Carbamylgruppe, stehen,
R₄ für eine gesättigte oder einfach, zweifach oder dreifach ungesättigte aliphatische Kette mit mindestens 12 Kohlenstoffatomen steht, und
n eine ganze Zahl von 1 bis 4 ist.

4. Lipo-Oligosaccharid nach Anspruch 3, dadurch **gekennzeichnet**, daß G für:
bei R. meliloti ein N-Acetyl-D-glucosamin-6-sulfat,
bei R. leguminosarum b.v. viciae ein N-Acetyl-D-glucosamin,
steht.

5. Lipo-Oligosaccharid nach einem der Ansprüche 3 oder 4, dadurch **gekennzeichnet**, daß es der nachstehenden Formel (II) entspricht: 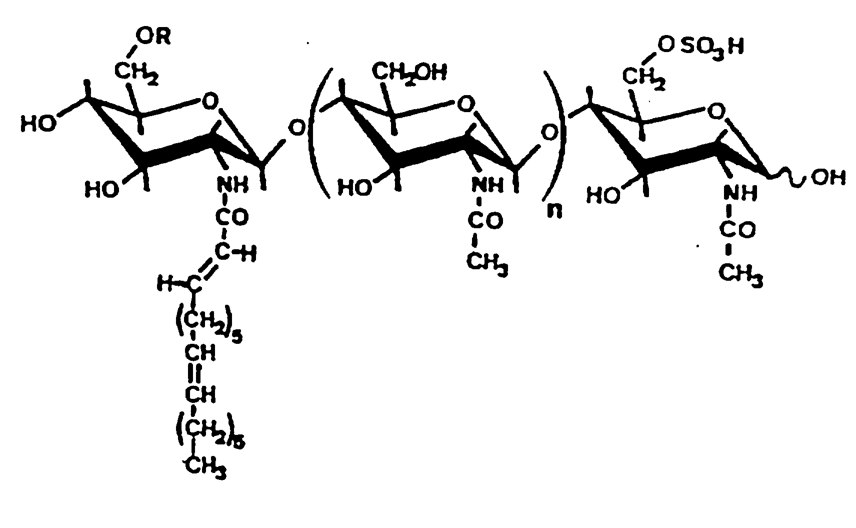 worin R für H oder CH₃CO- steht und n gleich 2 oder 3 ist.

6. Verfahren zur Herstellung eines Lipo-Oligosaccharids nach einem der Ansprüche 3 bis 5 durch Reinigung, dadurch **gekennzeichnet**, daß man in einer ersten Stufe ein rekombinantes Plasmid, resultierend aus der Klonierung in einem Plasmid, das sich in einem Knöllchenbakterium oder in einem anderen geeigneten Bakterium replizieren kann, (1) entweder eines Fragments, das gemeinsame und spezifische nod-Gene sowie Regulatorgene eines gegebenen Knöllchenbakteriums enthält, oder (2) von Regulatorgenen der Expression der nod-Gene herstellt, die Knöllchenbakterien oder ein geeignetes anderes Bakterium anschließend durch Einschleusen des genannten rekombinanten Plasmids modifiziert, um einen mutierten Nod-Faktoren stark überproduzierenden Stamm zu erhalten, und daß man den genannten mutierten Stamm in einem geeigneten Kulturmedium kultiviert und in einer zweiten Stufe den Überstand der Kultur gewinnt, den man durch Extraktion mit einem geeigneten niederen Alkohol oder durch geeignete Fest-Flüssig-Extraktion, gefolgt von einer Umkehrphasen-HPLC-Chromatographie des Extraktionsrückstands, einer Gelpermeations-Chromatographie, einer Ionenaustausch-Chromatographie und einer analytischen Unkehrphasen-HPLC-Chromatographie reinigt.

7. Verfahren nach Anspruch 6, dadurch **gekennzeichnet**, daß man das den Nod-Faktor überproduzierende Plasmid in ein mutiertes Knöllchenbakterium, das keine Exopolysaccharide herstellt, einschleust.

8. Verfahren zur Herstellung von Lipo-Oligosacchariden nach einem der Ansprüche 3 bis 5, dadurch **gekennzeichnet**, daß man von einem Wildstamm von Knöllchenbakterien, der Nod-Faktoren reichlich produziert, ausgeht, den man in einem geeigneten Kulturmedium kultiviert, und anschließend den Kulturüberstand gewinnt, den man gemäß der zweiten Stufe des Verfahrens nach Anspruch 6 reinigt.

9. Mittel zur Behandlung von Pflanzen, dadurch **gekennzeichnet**, daß sein oder ein aktiver Bestandteil ein Lipo-Oligosaccharid nach einem der Ansprüche 3 bis 5 ist.

10. Mittel zur Behandlung von Pflanzen nach Anspruch 9, dadurch **gekennzeichnet,** daß das Lipo-Oligosaccharid in einer Konzentration von 10⁻⁶ M bis 10⁻¹⁴ M vorliegt.

## Claims

1. Use of a lipo-oligosaccharide or a mixture of lipo-oligosaccharides, comprising a hexosamine oligomer whose nonreducing terminal hexosamine is N-substituted with a fatty acid as a plant-specific symbiotic signal to promote Rhizobiaceae-leguminosae symbiosis.

2. Use according to Claim 1, characterized in that the nonreducing terminal glucosamine of the N-acetylglucosamine oligomer is N-substituted with a fatty acid comprising not less than 12 carbon atoms.

3. Lipo-oligosaccharide characterized in that it is of the general formula I below: 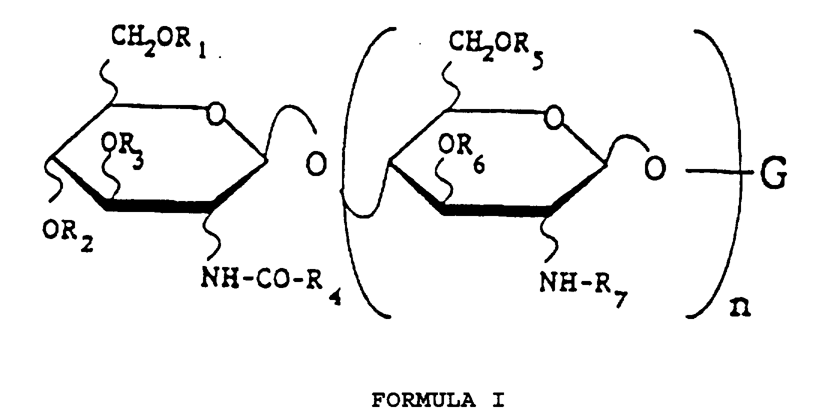 in which:
- G is a hexosamine which is variously substituted, for example, with an acetyl group on the nitrogen, a sulphate group, an acetyl group and/or an ether group on an oxygen,
- R₁, R₂, R₃, R₅, R₆ and R₇, which may be identical or different, represent H, CH₃CO-, CₓH_{y}CO- where x is an integer between 0 and 17, and y is an integer between 1 and 35, or any other acyl group such as for example a carbamyl
- R₄ represents a saturated aliphatic chain or a mono-, di- or triunsaturated aliphatic chain containing not less than 12 carbon atoms, and
- n is an integer between 1 and 4.

4. Lipo-oligosaccharide according to Claim 3, characterized in that G represents:
. in *R. meliloti*, an N-acetyl-D-glucosamine 6-sulphate
. in *R. leguminosarum b.v. viciae,* an N-acetyl-D-glucosamine.

5. Lipo-oligosaccharide according to either of Claims 3 and 4, characterized in that it is of the formula (II) below: 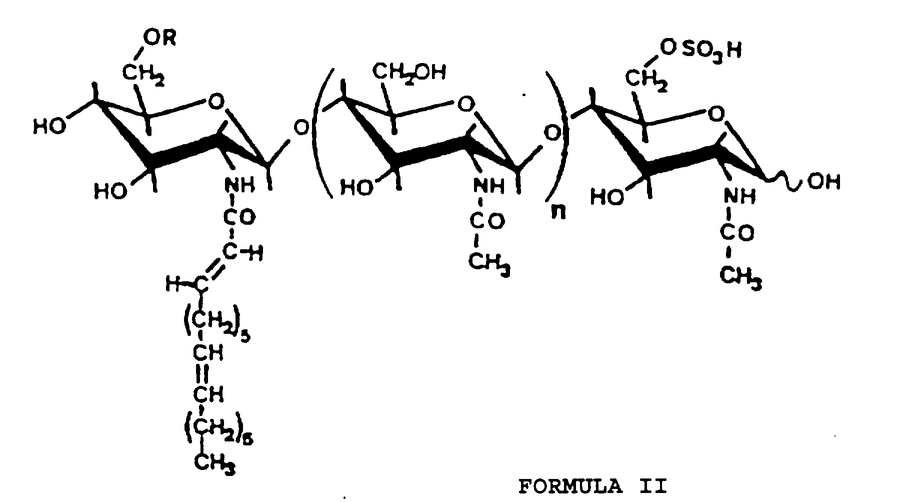 in which R represents H or CH₃CO- and n is equal to 2 or 3.

6. Process for producing a lipo-oligosaccharide according to any one of Claims 3 to 5, by purification, characterized in that during a first stage, a recombinant plasmid is produced which results from the cloning, into a plasmid capable of replicating in a Rhizobiaceae bacterium or any other appropriate bacterium, (1) either of a fragment which contains common and specific nod genes as well as the regulatory genes of a given Rhizobiaceae bacterium, or (2) of the regulatory genes for the expression of the nod genes. The Rhizobiaceae bacteria or any other appropriate bacteria are then modified by introducing the said recombinant plasmid in order to obtain a mutant strain which highly overproduces Nod factors and the said mutant strain is cultured in an appropriate culture medium; during a second stage, the culture supernatant is recovered and it is purified by extraction with an appropriate lower alcohol, or by solid-liquid extraction followed by a reversed-phase HPLC chromatography of the extraction residue, a gel permeation chromatography, an ion-exchange chromatography and a reversed-phase analytical HPLC chromatography.

7. Process according to Claim 6, characterized in that the Nod factor-overproducing plasmid is introduced into a non-exopolysaccharide-producing mutant Rhizobiaceae bacterium.

8. Process for producing lipo-oligosaccharides according to any one of Claims 3 to 5, characterized in that a wild strain of Rhizobiaceae bacteria, which is highly productive of Nod factors, is used as starting material, the said strain being cultured in an appropriate culture medium, after which the culture supernatant is recovered and purified in accordance with the 2nd stage of the process according to Claim 6.

9. Plant treatment agent characterized in that its or an active constituent is a lipo-oligosaccharide according to any one of Claims 3 to 5.

10. Plant treatment agent according to Claim 9, characterized in that the said lipo-oligosaccharide is present at a concentration of between 10⁻⁶ M and 10⁻¹⁴ M.
